# EUROPEAN PATENT SPECIFICATION

(11) **EP 0 470 246 B1**
(45) Date of publication and mention of the grant of the patent: **28.06.1995**
(21) Application number: 91906591.2
(22) Date of filing: 19.02.1991
(51) Int. Cl.: A61F 2/04, A61F 2/06, A61M 31/00, A61L 27/00, A61K 9/22

(54) **INTRALUMENAL DRUG ELUTING PROSTHESIS**
INTRALUMINALE PROTHESE MIT WIRKSTOFFELUIERUNG
PROTHESE POUR L'ELUTION INTRALUMINALE D'UN MEDICAMENT

(30) Priority: 28.02.1990 US 486580
(43) Date of publication of application: 12.02.1992
(73) Proprietor: MEDTRONIC, INC., Minneapolis, Minnesota 55432-3576 (US)
(72) Inventor: WOLFF, Rodney, G., Maple Grove, MN 55369 (US); HULL, Vincent, W., Fridley, MN 55432 (US)
(74) Representative: Tomlinson, Kerry John
(86) International application number: US9101097
(87) International publication number: WO9112779

(56) References cited:
- WO-A-89/03232
- WO-A-90/13332
- GB-A- 2 153 235
- US-A- 4 678 466
- US-A- 4 886 062

## Description

This invention relates to drug delivering intravascular stents for combatting restenosis of vascular lumens.

Restenosis is defined as the enclosure of a previously dilated, ablated, or lased peripheral or coronary vessel. It occurs at a rate of 20-50% for each of these procedures and is dependent on a number of variables (eg. vessel location and lesion length). Restenosis may begin immediately following an angioplasty procedure, but ceases at the end of approximately six months. There is not a current therapeutic procedure that has been proven to significantly reduce this restenosis rate.

A recent technology that has been developed that addresses the problem of restenosis is that of intravascular stents. Typical stent structures are described for example by Wiktor in US-A-4886062.

Stents are devices that are permanently implanted (expanded) in coronary and peripheral vessels. The goal of these stents is to provide a long-term "scaffolding" or support for the diseased (stenosed) vessels. The theory being that if you can support the vessel from the inside, the vessel will not close down or restenose. Unfortunately, initial data from clinical stent implants shows that metallic stent structures do not significantly reduce the amount of restenosis.

Many pharmacologic (biochemical) attempts have been made to reduce the amount of restenosis. All of these attempts have dealt with the systemic delivery of drugs via oral or intravascular introduction. Very limited success has been achieved with this systemic approach.

For drug delivery, it has been recognized for a long period of time that pills and injections may not be the best mode of administration. It is very difficult with these types of administration to get constant drug delivery. Through repeated doses, these drugs often cycle through concentration peaks and valleys, resulting in time periods of toxicity and ineffectiveness. Thus, localized drug treatment is warranted.

The present invention involves the use of localized physical and chemical inhibitors of vascular stenosis by the provision of a stent from which a chemical inhibitor is eluted.

The insertion into the vascular system of drug delivering structures has been described previously. However this has been either for immediate short term effect as for example in the anti-infection drug-releasing catheter tips of WO-89/03232 or has been to release into the blood agents intended to reach a susceptible downstream site, as for example with the drug delivery devices of US-A-4678466. The use of structures to provide localized inhibition both physically and chemically of vascular restenosis has not previously been envisaged.

Thus, viewed from one aspect, the invention provides an intravascular stent having means for the fixing thereof to an interior surface of a blood vessel, characterised in that said stent has at least a portion of the exterior surface thereof formed from a polymer incorporating a releasable vascular stenosis inhibiting drug and optionally provided with a drug diffusion limiting coating.

The stents of the invention may thus be inserted into a vascular lumen and fixed to the lumen wall adjacent an area needing treatment, eg. a part of the vascular system which may restenose.

The stents of the invention include at least one drug which will release, eg. at a controlled rate, to supply the drug where needed without the overkill of systemic delivery. The stents may be completely biodegradable or may be bioabsorbable in whole or in part such that they will be completely incorporated into the lumen wall as a result of tissue growth, i.e. endothelialization. Alternatively, the stent may be biostable in which case the drug is diffused out from the biostable materials in which it is incorporated.

The stent generally comprises a flexible tubular body which is fixed against the lumen walls by a mechanical action. The device should not cause an appreciable reduction in the lumen cross-section where inserted. Conventional stent designs which provide an expansion of the vessel are suitable, though not reguired. In all cases, the stents of the invention reguire the presence of an elutable drug compounded into the device itself. With conventional metal stents, the invention requires a drug-carrying coating overlying at least a portion of the metal.

The drugs in the stent may be of any type which would be useful in treating the lumen to contact stenosis. In order to prevent restenosis in blood vessels, migration and subsequent proliferation of smooth muscle cells must be checked. Platelet aggregation and adhesion can be controlled with antiplatelets and anticoagulants. Growth factor and receptor blockers and antagonists may be used to limit the normal repair response.

The current invention contemplates the usage of a stent which elutes drugs locally to treat a lumen in need of repair. Controlled release, via a bioabsorbable polymer, offers to maintain the drug level within the desired therapeutic range for the duration of the treatment. When "stent" is referred to herein, it may include the classical definition of stents as they are used in intravascular applications. "Stent" used herein also includes any prosthesis which may be inserted and held where desired in a vascular lumen. It includes, but is not limited to, structures such as those shown and described in US-A-4886062 (Wiktor).

A very simple definition of restenosis is given above. As a complement to this definition, there are several more clinical definitions. Several of these definitions are listed below:
1. Loss of at least 50% of the initial gain achieved in angioplasty.
2. Decrease of at least 30% in the lumen diameter compared to post-angioplasty result.
3. A return to within 10% of the pre-angioplasty diameter stenosis.
4. An immediate post angioplasty diameter stenosis of less than 50% that increases to 70% or greater at follow-up.
6. Deterioration of 0.72 mm in minimal luminal diameter or greater from post-angioplasty to follow-up.
7. As for 6, but with a deterioration of 0.5 mm.

These definitions are used by cardiologists to clinically (angiographically) define restenosis.

Several hypotheses exist on why and how restenosis occurs. The current, most widely accepted explanation is that restenosis is a natural healing process in response to the arterial injury that occurs during all types of angioplasty procedures. This very complex healing process results in intimal hyperplasia, more specifically migration and proliferation of medial smooth muscle cells (SMC). The problem associated with this arterial healing process is that in some instances, it does not shut off. The artery continues to "heal" until it becomes occluded. It should be noted that restenosis is not a re-deposition of the plaquelike cholesterol material that originally occluded the artery.

The following is a possible scenario for restenosis according to the vessel healing hypothesis. Successful angioplasty of stenotic lesions produces cracking of the plaque, dissection into the media, denudation and destruction of endothelial cells, exposure of thrombogenic collagens, released tissue thromboplastin, and an increased loss of protacyclin production. All of these lead to the aggregation of active platelets.

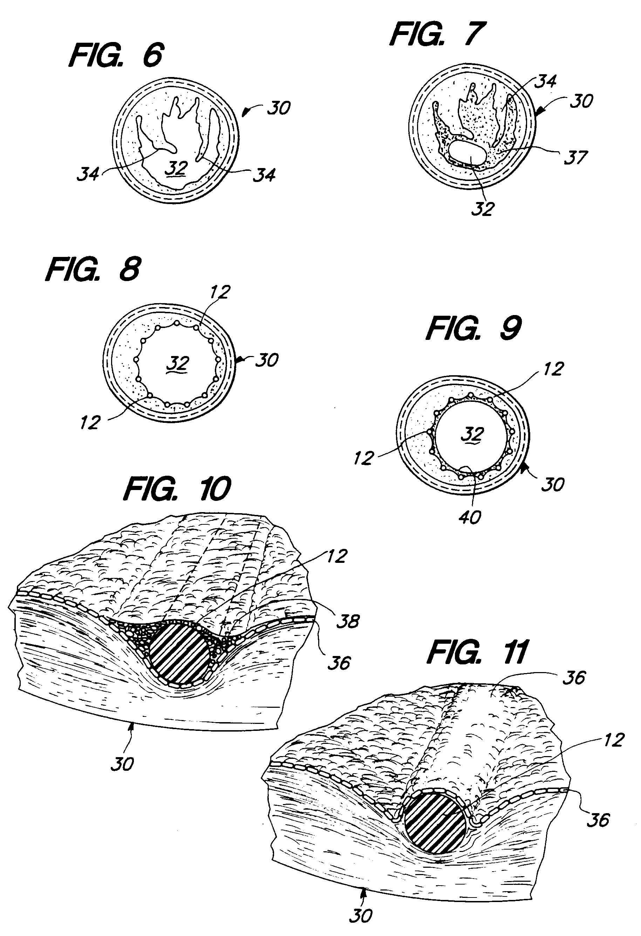

Figs. 6 and 7 show a typical blood vessel 30 in cross-section after angioplasty procedures and showing the interior 32 of the lumen. In Fig. 6 the interior of the lumen is rough and includes intimal flaps 34. Damage causes healing with deposition of platelets, fibrin formation and proliferation of neointima 37 which as shown in Fig. 7 significantly reduces the interior of the lumen.

Activated platelets release several mitogens including platelet derived growth factor (PDGF), epidermal growth factor, and transforming growth factor. PDGF has both mitogenic and chemotactic properties, and thus may induce both migration of SMC from the medial layer to the intimal layer as well as proliferation (intimal hyperplasia). PDGF causes SMC proliferation by binding to specific PDGF receptors. Once the PDGF is bound to the receptors, deoxyribose nucleic acid (DNA) synthesis occurs and new cells are replicated. Minor endothelial injury may cause platelet adhesion and activation with the resultant release of PDGF. Thus, even the deposition of a monolayer of platelets may be sufficient to induce SMC proliferation.

Deeper arterial injury which is sometimes associated with complex stenotic lesions leads to more extensive platelet deposition and activation which may cause an even greater availability of the mitogenic factors. Thus, increased SMC proliferation and intimal hyperplasia. Arterial injury from angioplasty may result in release of PDGF-like compounds from not only platelets but also macrophages, monocytes, endothelial cells, or SMC's themselves.

Activated SMC from human atheroma or following experimental arterial injury secrete PDGF-like molecules which appears to lead to self perpetuation of SMC proliferation by the release of their own PDGF-like substances. Thus, any or all of the cells which can secrete PDGF related substances (platelets, macrophages, monocytes, endothelia, and smooth muscle cells) may contribute to the cascading effect of restenosis after angioplasty.

The previous restenosis scenario resulted from normal angioplasty procedures. During balloon angioplasty if the balloon is undersized or not totally inflated and the plaque cracking and extensive endothelial denudation does not occur the lesion would restenose. Rheologic factors contribute as well to the interaction between platelets and the arterial wall. Residual stenosis, resulting from inadequate balloon expansion, produces a high local shear rate and enhances plateletdeposition and activation. These stenoses may be important as a stimulus for some proliferation through enhanced platelet deposition and secretion of growth factors. This hypothesis correlates with the increased incidence of restenosis in patients with high-grade residual stenoses or transtenotic gradients.

In order to prevent restenosis, one must stop the proliferation of smooth muscle cells. As stated earlier, this is a biochemical process which cannot be treated mechanically. Several hypotheses exist on how to biochemically stop restenosis. Some of which are:
1. Reduce the adhesion and aggregation of the platelets at the arterial injury site.
2. Block the expression of the growth factors and their receptors.
3. Develop competitive antagonists of the above growth factors.
4. Interfere with the receptor signaling in the responsive cell.
5. Find a "natural" inhibitor of smooth muscle proliferation.

Item #1 is directly related to the formation of thrombus, a major problem with all types of angioplasty procedures. Items #2, #3, and #4 are closely related. They deal with blocking restenosis during the massive cell migration and replication cycle. Unlike item #1, these items address the growth factors that are produced from sources other than platelets. Item #5 is listed to address the question, why don't the 50-80% of the people who don't restenose, restenose. There may be some type of natural inhibitor that these people produce that stops the proliferation of smooth muscle cells.

There are at least two different ways to prevent the adhesion and aggregation of platelets. One method is to use an antiplatelet and another is to use an anticoagulant.

Antiplatelet drugs include drugs such as aspirin and dipyridamole. Aspirin is classified as an analgesic, antipyretic, anti-inflammatory, antiplatelet drug. It has been clinically tested and proven to reduce the risk of sudden death and/or non-fatal reinfarction in post myocardial infarction (heart attack) patients. The proposed mechanism of how aspirin works, relates directly to the platelets. It somehow blocks the platelets, restricting coagulation. This prevents the cascading platelet aggregation found in thrombus and restenosis. Aspirin is therefore a possible restenosis inhibitor. Dipyridamole is a drug similar to aspirin, in that is has anti-platelet characteristics. Dypridimole is also classified as a coronary vasodilator. It increases coronary blood flow by primary selective dilatation of the coronary arteries without altering systemic blood pressure or blood flow in peripheral arteries. These vasodilation characteristics are thought to be possibly beneficial for restenosis prevention.

Anticoagulant drugs include Heparin, Coumadin, Protamine, and Hirudin. Heparin is the most common anticoagulant used today. Heparin, in one form or another, is used in virtually every angioplasty procedure performed. All four of these drugs function as an anticoagulant by preventing the production of thrombin, a binding agent which causes blood to clot. This too, may reduce the cascading effect of platelet aggregation at the lesion site, thus possibly reducing restenosis. The use of Protamine in the presence of Heparin causes the Protamine to function as a Heparin antagonist, blocking the effect of the Heparin. Protamine, however, used alone, acts as an anticoagulant. Hirudin is singled out because it is not normally found in the human body. Hirudin is a drug that is found in the salivary glands of leeches. It is a very concentrated anticoagulant that functions in the same manner as Heparin, Coumadin, and Protamine.

There are several types of drugs that interrupt cell replication. Antimitotics (cytotoxic agents) work directly to prevent cell mitosis (replication), whereas antimetabolites prevent deoxyribose nucleic acid (DNA) synthesis, thus preventing replication. The action of the antimitotics and antimetabolites are so similar, they will be grouped into one category. This category will be known as the anti-replicate drugs.

Anti-replicate drugs include among others: Methotrexate, Azathioprine, Vincristine, VinBlastine, Fluorouracil, Adriamycin, and Mutamycin. The target systemic molarity desired with methotrexate is on the order of 10⁻⁶ M with a range of between 10⁻³ to 10⁻⁸ Molar. Locally, the molarity of the drug may be highly variable, which is one of the great disadvantages in systemic administration of the drug. When drugs are delivered locally via the prosthesis of the invention, they may be at therapeutic levels at the diseased site while at the lower limits of detectability in the bloodstream. So little drug is required for effective local treatment of a lumen that the drug may not be detectable in blood samples.

If the restenosis process ranges from immediately after injury to about 4 months later, then the generalized elution rates contemplated are that the drug should start to be released immediately after the stent is secured to the lumen wall to lessen cell proliferation. The drug should then continue to elute for about four months in total.

Complex systems of drugs may be carried by the stent. An anticoagulant or antiplatelet may be included on the outermost surface of the device in order to elute off very quickly for the first several weeks. Antireplicates can be formulated into the device to continue to elute later, when in contact with non-blood cells after neointima overgrowth has surrounded the device. This usually occurs in about two weeks. The drug elution rate does not need to be uniform, and may be tailored to fit the need of the patient.

The current invention contemplates the usage of a stent which elutes drugs locally to treat a vascular (blood vessel) lumen in need of repair.

Embodiments of the invention are hereafter described with specific reference being made to the accompanying drawings, in which:
FIG. 1 is a greatly enlarged side view of an intralumenal drug-eluting stent of the invention;
FIG. 2 is a greatly enlarged side view of an alternative embodiment of the stent of the invention;
FIG. 3A is a greatly enlarged fragment of the embodiment of Fig. 1;
FIG. 3B is a greatly enlarged fragment of the embodiment of Fig. 1 in which two layers of polymer are present, each having a different drug;
FIG. 4 is a greatly enlarged fragment of the embodiment of Fig. 2;
FIG. 5 is a greatly enlarged microscopic fragmentary detail of drug shown eluting from the porous structure of a filament or filament coating in a stent into tissue or the vessel lumen;
FIG. 6 is a greatly enlarged cross-section of a blood vessel showing plaque profile immediately post-balloon catheter dilation procedure;
FIG. 7 is a greatly enlarged cross-section of the subject of Fig. 6 at a later date showing restenosis;
FIG. 8 is a greatly enlarged cross-section of a blood vessel showing plaque-stent profile immediately post-stent implant procedure;
FIG. 9 is a greatly enlarged cross-section of the subject of Fig. 8 after ingrowth has occurred;
FIG. 10 is a greatly enlarged fragmentary perspective view of a blood vessel wall and stent filament of Figs. 1 and 3 immediately after implantation;
FIG. 11 is a greatly enlarged fragmentary perspective view of the subject of Fig. 10 after about one month;
FIG. 12 is a greatly enlarged fragment of a loose weave of stent filaments;
FIG. 13 is a greatly enlarged fragment of a coated metal filament in a loose weave;
FIG. 14 is a greatly enlarged fragment of a melted junction weave of stent filaments in a loose weave;
FIG. 15 is a greatly enlarged fragment of a kinked junction weave of stent filaments;
FIG. 16 is a greatly enlarged fragment of multi strand weave of stent filaments; and
FIG. 17 is an alternative embodiment to Fig 16, in which the strands are not woven.

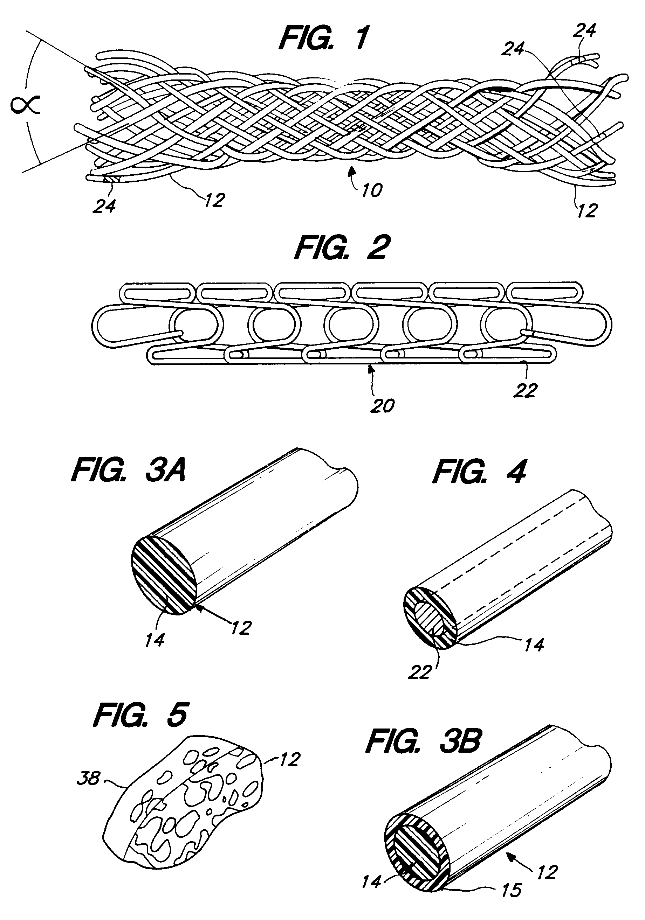

Figs. 1 through 17 show features of some of the stent which may be used to carry and elute restenosis limiting-drugs.

The current preferred configuration of stent 10 consists of a single filar, monofilament braided mesh design as shown in Fig. 1. There are sixteen filaments 12, eight of which are wound in one helical direction, with the remaining eight being wound in the opposite direction. The stent 10 is self-expanding to a predetermined diameter. The profile (diameter) of the stent 10 can be easily reduced by pulling the stent 10 longitudinally. In this reduced profile configuration, the stent 10 can be loaded into a catheter for delivery into the vessel.

The stent 20 shown in Figures 2 and 4 is a metallic malleable design which may be forced against a lumen wall by a balloon catheter which fixes it into position. The exterior surface of the metal filaments 22 would include a coating 14 with a drug-eluting polymer described previously. The polymer may be biostable or bioabsorbable. If biostable, the drug would diffuse out of the polymer.

The variations of design shown in the embodiments of Figs. 1 and 2 show that the stent of the invention must be secured against a lumen wall and must carry a drug-eluting polymer.

There are many variables in the design of stent 10. The angle (α) of the filaments 12 is a major variable. The angle a can vary from 0 degrees to 180 degrees. The design in the Figures is based on an angle in the 60 degree to 90 degree range.

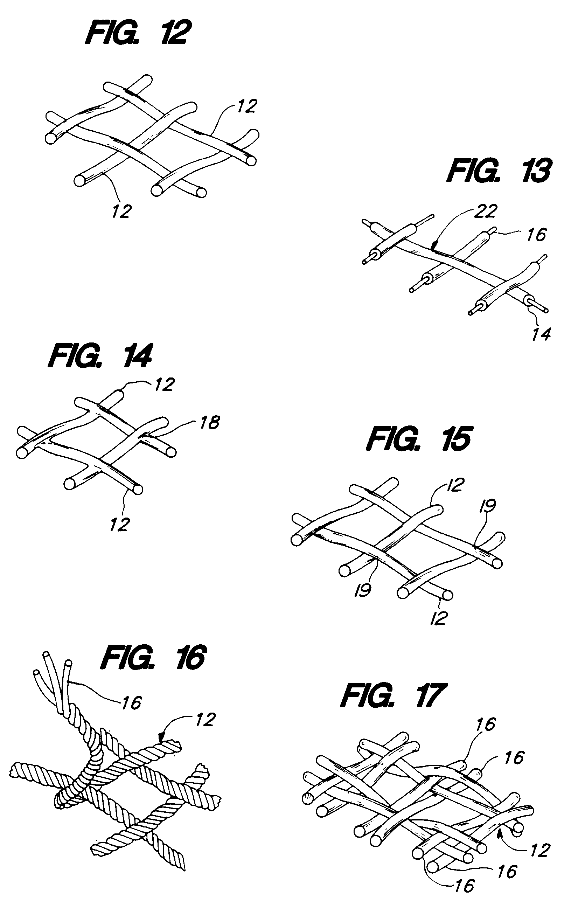

There are many options for fabricating the drug eluting stents. One option is to have all sixteen filaments be drug eluting. Or, you could have any number of filaments up to sixteen degrade and elute drugs. Another option is to have a multi-filar stent. Instead of a single filament braided into the stent, it is possible to have two, three, or even four strands 16 braided to form a filament 12 as shown in Fig. 16. This would create a stent with much greater expansile force, but would also have much more material in the surface area. This is a common tradeoff in stent design. Similar to the single-filar design, the multi-filar form shown in Fig. 16 could have varying numbers of strands 16 that are drug eluting. Figs. 16 and 17 show that the multi-filar design may be braided or unbraided. One, two, three, or four of the filaments could be impregnated with a drug and biodegradably elute. Alternatively, the polymer may be biostable which allows for diffusion of the drug without degradation.

The stent 10 of Fig. 1 consists of a wound braided mesh which is self-expanding to a predetermined diameter and whose profile diameter can be greatly reduced for catheter introduction. The radial expansile force increases with diameter to the point of the self-expanded diameter limit, at which point the angle between the filaments and the longitudinal axis is a maximum. Figures 12 through 15 show alternative construction techniques to alter the radial expansive force. Figure 12 shows the filaments 12 being woven without any connection. Figure 13 is similar except the filament 22 is formed with a metal core 16 and a coating 14. In Fig. 14 the individual filaments 12 are shown with a bonded juncture 18. The bonding at the junctures 18 prevents the individual filaments 12 from sliding relative to each other, which improves the radial strength. The mechanically kinked junction 19 shown in Fig. 15 also limits the sliding of the filaments to change the radial strength. A heated platen press may be pressed against the wound stent while still on the forming mandrel to form the kinks. Higher temperatures may be used to form the melted junctures 18.

The devices may be made more visible under fluoroscopy and x-ray by incorporating radiopaque materials into marker bands 24 to the individual filaments 12 at the ends of the stent 10 as shown in Fig. 1. Such bands could help to locate the stent and assure proper placement and patency.

Controlled release, via a bioabsorbable polymer, offers to maintain the drug level within the desired therapeutic range for the duration of the treatment. In the case of stents, the prosthesis materials will maintain vessel support for at least two weeks or until incorporated into the vessel wall even with bioabsorbable, biodegradable polymer constructions.

Several polymeric compounds that are known to be bioabsorbable and hypothetically have the ability to be drug impregnated may be useful in prosthesis formation herein. These compounds include: poly-l-lactic acid/polyglycolic acid, polyanhydride, and polyphosphate ester. A brief description of each is given below.

Poly-l-lactic acid/polyglycolic acid has been used for many years in the area of bioabsorbable sutures. It is currently available in many forms, eg. crystals, fibers, blocks and plates. These compounds degrade into non-toxic lactic and glycolic acids. There are, however, several problems with this compound. The degradation artifacts (lactic acid and glycolic acid) are slightly acidic. The acidicity causes minor inflammation in the tissues as the polymer degrades. This same inflammation could be very detrimental in coronary and peripheral arteries, i.e. vessel occlusion. Another problem associated with this polymer is the ability to control and predict the degradation behavior. It is not possible for the biochemist to safely predict degradation time. This would be very detrimental for a drug delivery device.

Other compounds which could be used are the polyanhydrides. They are currently being used with several chemotherapy drugs for the treatment of cancerous tumors. These drugs are compounded in the polymer which is molded into a cube-like structure and surgically implanted at the tumor site.

Polyanhydrides have weaknesses in their mechanical properties, due to low molecular weights. This drawback makes them difficult to process into a filament form. Also, polyanhydrides have poor solubility, making characterization and fabrication difficult.

The compound which is preferred is a polyphosphate ester. Polyphosphate ester is a proprietary compound which is currently being developed by Dr. Ham Leong at John Hopkins University (JHU). Similar to the polyanhydrides, polyphosphate ester is being researched for the sole purpose of drug delivery. Unlike the polyanhydrides, the polyphosphate esters have high molecular weights (600,000 average), yielding attractive mechanical properties. This high molecular weight leads to transparency, and film and fiber properties. It has also been observed that the phosphorus-carbon-oxygen plasticizing effect, which lowers the glass transition temperature, makes the polymer desirable for fabrication.

The basic structure of polyphosphate ester monomer is shown below. 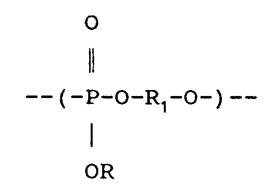
where P denotes Phosphorus,
O denotes Oxygen,
and R and R₁ are functional groups.

Reaction with water leads to the breakdown of this compound into monomeric phosphates (phosphoric acid) and diols (see below). 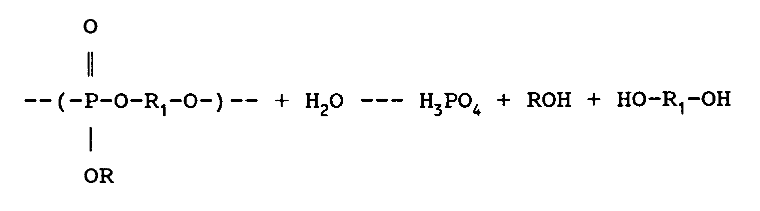
It is the hydrolytic instability of the phosphorus ester bond which makes this polymer attractive for controlled drug release applications. A wide range of controllable degradation rates can be obtained by adjusting the hydrophobicities of the backbones of the polymers and yet assure biodegradability.

The functional side groups allow for the chemical linkage of drug molecules to the polymer. This is shown below. 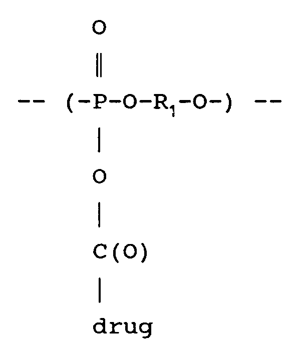
The drug may also be incorporated into the backbone of the polymer. 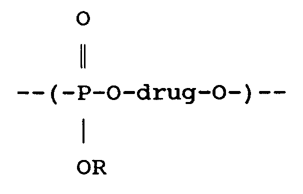
In summary, the highly hydrolytically reactive phosphorus ester bond, the favorable physical properties, and the versatile chemical structure make the polyphosphate esters a superior drug delivery system for stents.

Figs. 3A and 3B show that the filaments 12 may be made from one or several layers of polymer. In Fig. 3A only a single polymer is present to carry the drugs. In Fig. 3B a second layer of polymer 15 is shown. That layer 15 may be a simple barrier which limits diffusion of drugs in the polymer 14. In that event, the smaller molecules could elute out immediately, while larger compounds would not elute until later when the layer 15 has biodegraded. Alternatively, layer 15 may include a different drug incorporated therein from that found in layer 14. The barrier coating 15 could be as simple as a silicone or polyurethane.

The stent is inserted into the vascular lumen wherever needed as per the usual procedure for stents. The device is fixed into place either by radial expansion in devices such as shown in Fig. 1 or it is deformed by a balloon catheter in the case of devices in accordance with Fig. 2.

Figures 8 through 11 show the placement and effects of the drug-eluting stents of the invention. The stent tacks up any intimal flaps and tears caused by any prior ballooning. The initial deposition of platelets and subsequent thrombus formation 38 is controlled and minimized by the stent design and the elution of drug which limits platelet aggregation and other immediate repair responses described previously. Localized thrombus formations in the areas of cracked and roughened plaques and newly exposed underlying collagen and fibro-muscular tissues is also decreased. This results in limited but quick neointima formation 40 and intimal proliferation over individual stent filaments progressing to mature endothelial lining. Long term restenosis is therefore limited. Elution of the antireplicates alone or in conjunction with the initial elution of anti-coagulants can also limit the restenosis which occurs in the natural healing process.

The use of stenosis-inhibiting drugs for the manufacture of stents according to the invention forms a further aspect of the invention.

## Claims

1. An intravascular stent (10) having means (12) for the fixing thereof to an interior surface of a blood vessel, characterised in that said stent has at least a portion of the exterior surface thereof formed from a polymer incorporating a releasable vascular stenosis inhibiting drug and optionally provided with a drug diffusion limiting coating.

2. A stent as claimed in claim 1 comprising at least two separate layers of polymer each having a different said drug therein.

3. A stent as claimed in either of claims 1 and 2 wherein said drug is selected from antiplatelet anticoagulant, antiinflammatory and antimetabolite drugs.

4. A stent as claimed in any one of claims 1 to 3 wherein said polymer is bioabsorbable.

5. A stent as claimed in any one of claims 1 to 4 being a self expanding stent.

6. A stent as claimed in any one of claims 1 to 5 having a body portion composed of a plurality of thread elements each woven in a helix configuration along the centre line of said body as a common axis, said body including elements wound in opposing helical directions.

7. A stent as claimed in claim 6 radially extendable under axial compression of said body portion.

8. A stent as claimed in any one of claims 1 to 7 wherein said polymer is in filamentary form.

9. A stent as claimed in any one of claims 1 to 8 wherein a said diffusion limiting coating is provided on said drug-incorporating polymer.

10. A stent as claimed in claim 9 wherein at least one said element includes an anticoagulant drug and at least one said element includes an antimetabolite drug.

11. The stent as claimed in any one of claims 1 to 4 comprising metal filaments provided with a coating of said drug-incorporating polymer.

12. The use of a vascular stenosis limiting drug in the manufacture of a polymeric composition for use in a method of combatting vascular stenosis by the topical administration of said drug to a vascular lumen from an intravascular stent according to any of claims 1 to 11.

13. Use as claimed in claim 12 wherein said drug is selected from antiplatelet drugs, anti-inflammatory drugs, antimetabolite drugs and combinations thereof.

14. Use as claimed in either one of claims 12 and 13 wherein said polymeric composition is bioabsorbable.

## Patentansprüche

1. Intravaskulärer Stent (10) mit Mitteln (12) zu dessen Fixierung an der Innenseite eines Blutgefäßes, dadurch gekennzeichnet, daß wenigstens ein Teil der Außenseite des Stent aus einem Polymer gebildet wird, in welchem ein freisetzbares, Gefäßstenose-inhibierendes Medikament enthalten ist, und welches gegebenenfalls mit einer die Diffusion des Medikaments limitierenden Beschichtung versehen ist.

2. Stent nach Anspruch 1, umfassend wenigstens zwei getrennte Polymerschichten, wobei jede davon ein anderes Medikament aufweist.

3. Stent nach einem der Ansprüche 1 und 2, worin das Medikament ausgewählt ist unter einem Antithrombocyten-Antikoagulans, antiinflammatorischen und Antimetabolit-Medikamenten.

4. Steht nach einem der Ansprüche 1 bis 3, wobei das Polymer biologisch absorbierbar ist.

5. Stent nach einem der Ansprüche 1 bis 4, nämlich ein selbstexpandierender Stent.

6. Stent nach einem der Ansprüche 1 bis 5, mit einem Körperabschnitt, zusammengesetzt aus einer Vielzahl fadenförmiger Elemente, wobei jedes davon helikal entlang der Mittelinie des Körpers als gemeinsamer Achse gewebt ist, und wobei der Körper Elemente enthalt, die in entgegengesetzter helikaler Richtung gewunden sind.

7. Stent nach Anspruch 6, radial dehnbar unter axialer Kompression des Körperabschnitts.

8. Stent nach einem der Ansprüche 1 bis 7, wobei das Polymer faserförmig ist.

9. Stent nach einem der Ansprüche 1 bis 8, worin die die Diffusion limitierende Beschichtung auf dem Polymer, welches das Medikamt enthält, ausgebildet ist.

10. Stent nach Anspruch 9, worin wenigstens eines der Elemente ein Antikoagulans und wenigstens eines der Elemente einen Antimetaboliten enthält.

11. Stent nach einem der Ansprüche 1 bis 4, umfassend Metallfilamente, welche mit einer Beschichtung des das Medikament enthaltenden Polymers versehen sind.

12. Verwendung eines Gefäßstenose-limitierenden Medikaments bei der Herstellung einer Polymerzusammensetzung für die Verwendung in einem Verfahren zur Bekämpfung von Gefäßstenose durch topische Verabreichung des Medikaments in das Gefäßlumen aus einem intravaskullären Stent gemäß einem der Ansprüche 1 bis 11.

13. Verwendung nach Anspruch 12, worin das Medikament ausgewählt ist unter Antitrombocyten-Medikamenten, antiinflammatorischen Medikamenten, Antimetabolit-Medikamenten und Kombinationen davon.

14. Verwendung nach einem der Ansprüche 12 und 13, worin die Polymerzusammensetzung biologisch absorbierbar ist.

## Revendications

1. Prothèse intravasculaire, couramment appelée "stent", (10) possédant des moyens (12) pour sa fixation à la surface interne d'un vaisseau sanguin, caractérisée en ce que ledit stent a au moins une portion de sa surface externe formée d'un polymère incorporant un médicament libérable inhibant la sténose vasculaire et pourvue facultativement d'un revêtement limitant la diffusion de médicament.

2. Stent selon la revendication 1, comprenant au moins deux couches séparées de polymère, ayant chacune un médicament différent à l'intérieur.

3. Stent selon l'une quelconque des revendications 1 et 2, dans lequel ledit médicament est sélectionné parmi des antiplaquettaires, anticoagulants, antiinflammatoires et antimétabolites.

4. Stent selon l'une quelconque des revendications 1 à 3, dans lequel ledit polymère est bioabsorbable.

5. Stent selon l'une quelconque des revendications 1 à 4, ayant la propriété d'être autoextensible.

6. Stent selon l'une quelconque des revendications 1 à 5, dont la partie corps est composée d'une pluralité d'éléments de fils, tissés chacun dans une configuration en hélice le long de la ligne de centre dudit corps comme axe commun, ledit corps comprenant des éléments enroulés dans des directions hélicoïdales opposées.

7. Stent selon la revendication 6, extensible de façon radiale sous la compression axiale de ladite partie corps.

8. Stent selon l'une quelconque des revendications 1 à 7, dans lequel ledit polymère est dans une forme filamentaire.

9. Stent selon l'une quelconque des revendications 1 à 8, dans lequel ledit revêtement limitant la diffusion est appliqué audit polymère incorporant un médicament.

10. Stent selon la revendication 9, dans lequel au moins un desdits éléments inclut un anticoagulant et au moins un desdits éléments inclut un antimétabolite.

11. Stent selon l'une quelconque des revendications 1 à 4, comprenant des filaments en métal pourvus d'un revêtement dudit polymère incorporant un médicament.

12. Utilisation d'un médicament limitant la sténose vasculaire dans la fabrication d'une composition de polymère pour utilisation dans une méthode de lutte contre la sténose vasculaire par l'administration topique dudit médicament à l'intérieur d'un vaisseau à partir d'un stent intravasculaire selon l'une quelconque des revendications 1 à 11.

13. Utilisation selon la revendication 12, dans laquelle ledit médicament est sélectionné parmi des antiplaquettaires, des antiinflammatoires, des antimétabolites et des combinaisons de ceux-ci.

14. Utilisation selon l'une quelconque des revendications 12 et 13, dans laquelle ladite composition de polymère est bioabsorbable.
